# FASCICULE DE BREVET EUROPEEN

(11) **EP 0 914 124 B1**
(45) Date de publication et mention de la délivrance du brevet: **21.01.2004**
(21) Numéro de dépôt: 97935593.0
(22) Date de dépôt: 21.07.1997
(51) Int. Cl.: A61K 31/435, A61K 31/44, A61P 7/02, A61P 9/10

(54) **COMPOSITION PHARMACEUTIQUE ANTITHROMBOTIQUE ET ANTIATHEROGENE COMPRENANT UN DERIVE DE THIENOPYRIDINE ET UN INHIBITEUR DE LA HMG-CoA-REDUCTASE**
ANTITHROMBOTISCHE ANTIATHEROSKLEROTISCHE PHARMAZEUTISCHE ZUSAMMENSETZUNG AUS EINEM THIENOPYRIDINDERIVAT UND EINEM HMG-COA-REDUKTASE-HEMMER
ANTITHROMBOTIC AND ANTIATHEROGENIC PHARMACEUTICAL COMPOSITION INCLUDING A THIENOPYRIDINE DERIVATIVE AND AN HMG-CoA-REDUCTASE INHIBITOR

(30) Priorité: 26.07.1996 FR 9609474
(43) Date de publication de la demande: 12.05.1999
(73) Titulaire: SANOFI-SYNTHELABO, 75635 Paris Cédex 13 (FR)
(72) Inventeur: DASTE, Georges, F-33200 Bordeaux (FR); HERBERT, Jean-Marc, F-31170 Tournefeuille (FR)
(74) Mandataire: Bernasconi, Jean Raymond
(86) Numéro de dépôt international: PCT/FR1997/001353
(87) Numéro de publication internationale: WO 1998/004259

(56) Documents cités:
- EP-A- 0 373 507
- EP-A- 0 472 449
- WO-A-95/11898
- WO-A-95/13063
- KOSUGI T. ET AL: "CHANGES OF PLATELET FUNCTION ON SIMULTANEOUS ADMINISTRATION OF TICLOPIDINE AND OKY-046" INTERNATIONAL JOURNAL OF TISSUE REACTIONS, vol. 13, no. 3, 1991, pages 124-129, XP000646530
- A. BERNAT ET AL: "potentiating effects of anticoagulants and antiplatelet agents on streptokinase-induced thrombolysis in the rabbit" FIBRINOLYSIS, vol. 7, no. 1, 1993, pages 23-30, XP000646523
- J. DAVIGNON: "prospects for drug therapy for hyperlipoproteinemia" DIABETE & METABOLISME, vol. 21, 1995, PARIS, pages 139-146, XP000618561

## Description

La présente invention concerne une composition pharmaceutique contenant. en tant que principe actif, une association d'un dérivé de thiénopyridine et d'un inhibiteur de la HMG-CoA réductase.

Plus particulièrement, la présente invention a pour objet une composition pharmaceutique contenant
(a) un dérivé de thiénopyridine de formule 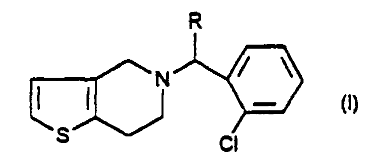 dans laquelle R est l'hydrogène ou un groupe (C₁-C₄)alcoxycarbonyle, ou un de ses sels pharmaceutiquement acceptables; et
(b) un inhibiteur de la HMG-CoA-réductase.

Il est connu que les dérivés de thiénopyridine de formule (I) sont des antiagrégants plaquettaires puissants, agissant par un mécanisme d'action qui les distinguent d'autres antiagrégants plaquettaires.

Ces composés, notamment la 5-(2-chlorobenzyl)-4,5,6,7-tétrahydrothiéno[3.2-c] pyridine de formule (I), R = hydrogène, ci-après désignée par sa Dénomination Commune internationale (DCI) "ticlopidine", utilisée sous forme de chlorhydrate, et le (+)-(S)-α-(2-chlorophényl)-4,5,6,7-tétrahydrothiéno[3,2-c] pyridinyl-5 acétate de méthyle, de formule (I), R = méthoxycarbonyle, ci-après désigné par sa DCI clopidogrel", utilisé sous forme d'hydrogénosulfate, sont des agents antithrombotiques remarquables (Gent *et al*., Lancet, 1989, 8649, 1215-1220 - J-M. Herbert *et al*., Cardiovasc. Drug Rev., 1993, *11*, 180-188).

Il a été maintenant trouvé qu'en associant à un dérivé de thiénopyridine de formule (1) un inhibiteur de la HMG-CoA réductase, on observe une potentialisation du pouvoir anti-athérogène des deux composants, plus particulièrement une activité additive et/ou synergique des deux principes actifs sur la prolifération des cellules musculaires lisses de l'artère de lapin.

Il a été également trouvé que l'association d'un dérivé de thiénopyridine de formule (I) et d'un inhibiteur de HMG-CoA réductase est pourvue d'une activité additive et/ou synergique des deux principes actifs dans un modèle animal de thrombose artérielle prédictifs d'une activité antithrombotique clinique préventive ou curative.

Ainsi, selon la présente invention, une dose thérapeutiquement efficace d'un dénvé de thiénopyndine de formule (I), ou d'un de ses sels pharmaceutiquement acceptables [Composant (a)], est associée à une dose thérapeutiquement efficace d'un inhibiteur de HMG-CoA réductase [Composant (b)] afin de préparer des compositions pharmaceutiques destinées à traiter ou à prévenir l'athérosclérose, la resténose post-angioplastie ou les complications thrombotiques en résultant.

Dans une unité de dosage contenant l'association d'une thiénopyridine de formule (I) et d'un inhibiteur de HMG-CoA réductase, la dose thérapeutiquement efficace du Composant (a) peut varier de 10 à 250 mg de principe actif (calculé en base libre ou en sel), alors que la dose thérapeutiquement efficace du Composant (b) peut varier de 2 à 50 mg de principe actif.

Selon la présente invention, le dérivé de thiénopyridine de formule (I) est choisi de préférence parmi la ticlopidine et les sels pharmaceutiquement acceptables. notamment le chlorhydrate de ticlopidine, le clopidogrel et les sels pharmaceutiquement acceptables, notamment l'hydrogénosulfate de clopidogrel.

Lorsque le Composant (a) dans une unité de dosage est le chlorhydrate de ticiopidine, la quantité de ce principe actif dans l'unité de dosage peut varier avantageusement de 100 à 250 mg, ladite quantité de principe actif étant de préférence de 150, 175, 200, 225 ou 250 mg par unité de dosage.

Lorsque, dans l'unité de dosage, le Composant (a) est l'hydrogénosulfate de clopidogrel, la quantité de ce principe actif dans l'unité de dosage peut varier avantageusement de 10 à 75 mg (calculés en base libre), ladite quantité de pnncipe actif étant de préférence de 25, 35, 50, 65 ou 75 mg en base libre par unité de dosage.

Selon la présente invention, l'inhibiteur de la HMG-CoA réductase est avantageusement un composé choisi parmi
(i) les dérivés de naphthalène de formule (II) 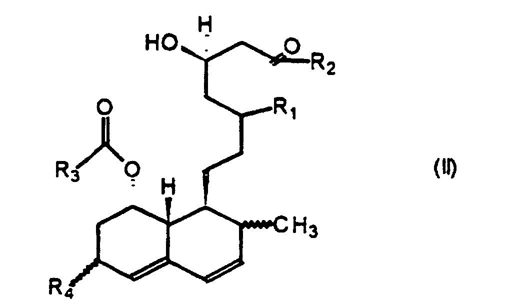 dans laquelle R₁ et R₂ sont un groupe hydroxy ou bien, ensemble forment un atome d'oxygène, R₃ est un groupe (C₁-C₁₀)alkyle, (C₃-C₁₀)cycloalkyle, (C₂-C₁₀)alkényle, phényle ou phényl(C₁-C₃)alkyle et R₄ est l'hydrogène ou un groupe méthyle ou hydroxyle;
(ii) les sels pharmaceutiquement acceptables des composés de formule (II) dans laquelle R₁ et R₂ sont hydroxy;
(iii) les dérivés de l'indole de formule (III) 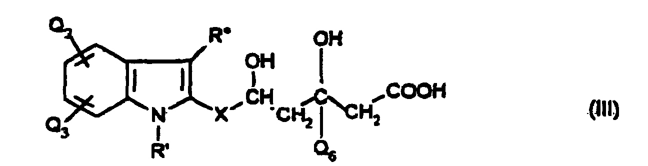 dans laquelle
   - un des R° et R' est un groupe de structure 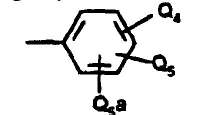
   dans laquelle Q₄ est un atome d'hydrogène, de chlore ou de fluor ou un groupe (C₁-C₄)alkyle, (C₁-C₄)alcoxy, (mais autre que t-butoxy), trifluorométhyle, phénoxy ou benzyloxy, Q₅ est un atome d'hydrogène, de chlore, de fluor ou un groupe phénoxy ou benzyloxy, Q₅ₐ est un atome d'hydrogène, de chlore, de fluor ou un groupe méthyle, éthyle, méthoxy ou éthoxy;
   et l'autre des R° et R' est un un groupe (C₁-C₆)alkyle primaire ou secondaire, (C₃-C₆)cycloalkyle, benzyle, 2-phényléthyle ou 3-phénylpropyle;
   - Q₂ est un atome d'hydrogène, de fluor, de chlore ou un groupe (C₁-C₄)alkyle, (C₃-C₆)cycloalkyle, (C₁-C₄)alcoxy (mais autre que t-butoxy), trifluorométhyle, phénoxy ou benzyloxy;
   - Q₃ est un atome d'hydrogène, de chlore, de fluor ou un groupe (C₁-C₃)alkyle, (C₁-C₃)alcoxy, phénoxy ou benzyloxy;
   - X est un groupe méthylène, éthylène ou 1,3-propylène;
   - Q₆ est un atome d'hydrogène ou un groupe (C₁-C₃)alkyle;
   avec la limitation que
   (1) Q₅ et Q₅ₐ sont l'hydrogène lorsque R₄ est l'hydrogène,
   (2) Q₅ₐ est l'hydrogène lorsque Q₅ est l'hydrogène,
   (3) Q₄ et Q₃ ne sont pas en même temps un groupe trifluorométhyle, phénoxy ou benzyloxy,
   (4) Q₃ est l'hydrogène lorsque Q₂ est l'hydrogène.
   (5) Q₂ et Q₃ ne sont pas en même temps un groupe trifluorométhyle, phénoxy ou benzyloxy;
(iv) les esters pharmaceutiquement acceptables des composés de formule (III),
(v) les sels pharmaceutiquement acceptables des composés de formule (III),
(vi) les δ-lactones des composés de formule (III),
(vii) les dérivés du tétrazole de formule (IV) 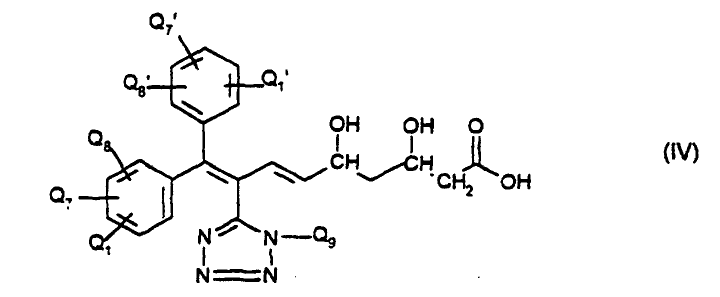 dans laquelle
   - Q₁ et Q₁' sont l'hydrogène, un halogène ou un groupe (C₁-C₄)alkyle, (C₁-C₄)-alcoxy ou trifluorométhyle;
   - Q₇, Q₇', Q₈ et Q₈' sont l'hydrogène, un halogène ou un groupe (C₁-C₄)alkyle, (C₁-C₄)alcoxy;
   - Q₉ est l'hydrogène ou un groupe (C₁-C₄)alkyle, (C₁-C₄)alcoxyalkyle ou (2-méthoxyéthoxy)méthyle;
(viii) les sels pharmaceutiquement acceptables des composés de formule (IV),
(ix) les δ-lactones des composés de formule (IV),
(x) les dérivés de pyridine de formule (V) 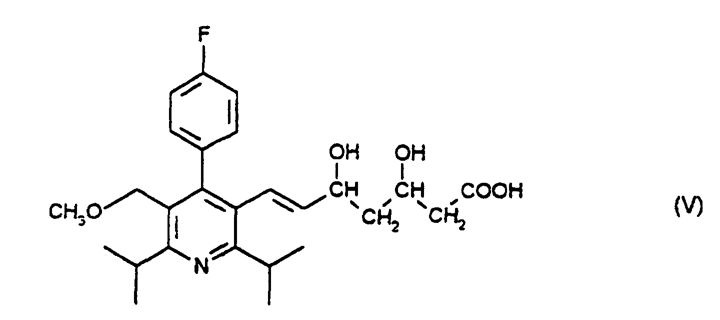
(xi) les sels pharmaceutiquement acceptables des composés de formule (V).
(xii) les δ-lactones des composés de formule (V),
(xiii) les dérivés du pyrrole de formule (VI) 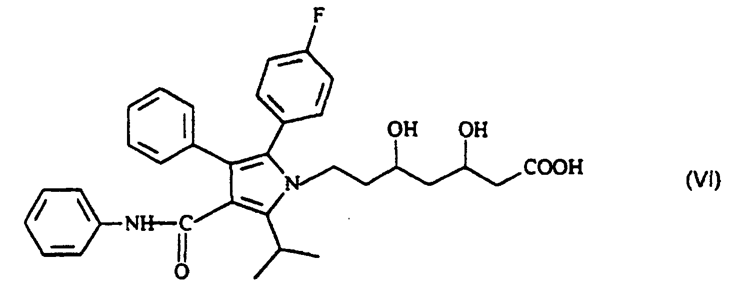
(xiv) les sels pharmaceutiquement acceptables des composés de formule (VI),
(xv) les δ-lactones des composés de formule (VI).

Les composés répondant aux formules (II) à (VI) possèdent au moins deux carbones chiraux, les composés (II) à (V) pouvant aussi être présents sous la forme cis ou trans. Le Composant (b) peut être choisi parmi les isomères des composés (II) à (VI) ou leurs mélanges.

Les composés (II) à (VI) sont décrits en littérature. Plus particulièrement, les dérivés de l'indole de formule (III) sont décrits dans WO 84/02131, les dérivés du tétrazole de formule (IV) sont décrits dans EP 658550, les dérivés de la pyridine de formule (V) sont décrits dans DE 4040026 et les dérivés du pyrrole de formule (VI) sont décrits dans EP 409281.

Les composés du naphtalène de formule (II), dans laquelle R₄ est l'hydrogène ou un groupe méthyle, sont décrits dans EP 33538, ou peuvent être préparés soit selon la méthode décrite dans ce document, soit par semisynthèse, soit, dans certains cas, par synthèse totale, par exemple dans le cas de la synthèse de la mévastatine (J. Am. Chem. Soc., 1981, 6538; ibid. 1982, 4251) ou de la lovastatine (Tetrahedron Letters. 1983, 24, 1811). La simvastatine est également décrite dans EP33538.

Les composés du naphtalène de formule (II), dans laquelle R₄ est l'hydroxyle sont décrits dans GB 2 077 264. Parmi ces composés, la pravastatine, utilisée sous forme de sel de sodium, constitue un Composant (b) particulièrement intéressant.

Parmi les indoles de formule (III), le composé ayant cette formule, dans laquelle R° est 4-fluorophényle, R' est isopropyle, X est éthylène et Q₂, Q₃ et Q₆ sont l'hydrogène, dans sa forme (E), racémique ou optiquement active et ses sels pharmaceutiquement acceptables constituent un Composant (b) particulièrement intéressant pour les compositions pharmaceutiques de la présente invention.

Parmi les dérivés du tétrazole de formule (IV), le composé ayant cette formule, dans laquelle Q₁ et Q₁' sont chacun un atome de fluor, Q₇, Q₇', Q₈ et Q₈' sont l'hydrogène et Q₉ est un groupe méthyle, dans sa forme (E), racémique ou optiquement active, de préférence (βR),(δS) et ses sels pharmaceutiquement acceptables, notamment avec un acide aminé, constituent également un Composant (b) particulièrement intéressant pour les compositions pharmaceutiques de la présente invention.

Le dérivé de pyridine de formule (V), dans sa forme (E), racémique ou optiquement active, de préférence (βR),(δS), ou cérivastatine, et ses sels pharmaceutiquement acceptables, constituent un autre Composant (b) intéressant pour les compositions pharmaceutiques de la présente invention.

La δ-lactone du dérivé du pyrrole de formule (VI), connu comme atorvastatine, constitue un Composant (b) intéressant pour les compositions pharmaceutiques de la présente invention.

Selon un aspect avantageux, les compositions de la présente invention contiennent le chlorhydrate de ticlopidine en tant que Composant (a) et la simvastatine ou la pravastatine sodique en tant que Composant (b). De préférence, de telles compositions renferment de 100 à 250 mg de chlorhydrate de ticlopidine et de 10 à 40 mg de simvastatine ou de pravastatine sodique.

Selon un autre aspect avantageux, les compositions de la présente invention contiennent l'hydrogénosulfate de clopidogrel en tant que Composant (a) et la simvastatine ou la pravastatine sodique en tant que Composant (b). De préférence. de telles compositions renferment de 10 à 75 mg (calculés en base libre) de clopidogrel hydrogénosulfate et de 10 à 40 mg de simvastatine ou pravastatine sodique.

Les associations de principes actifs selon l'invention ont fait l'objet d'études pharmacologiques.

Des essais ont été réalisés *vis-à-vis* du test de prolifération des cellules musculaires de la paroi vasculaire apparaissant consécutivement à une déendothélialisation de l'artère carotide de lapins. Brièvement, des lapins néo-zélandais de 2,5-3 kg ont été nourris avec un aliment synthétique contenant 2% de cholestérol et 6% d'huile d'arachide. Les animaux ont été traités par voie orale par du Clopidogrel (5 mg/kg/j). Les animaux ont été traités simultanément par voie orale par la simvastatine (5 mg/kg/j). Les composés ont été administrés 2 jours avant la lésion de l'endothélium et quotidiennement pendant 2 semaines. La prolifération myowtimale de la caroide de lapin a été induite par déssication à l'air comme décrit selon la méthode décrite précédemment (Fishman *et al*., Lab. Invest., 1975, 32, 339-347; Herbert *et al*., 1993, 13, 1171-1179). Les animaux ont été anesthésiés par voie intraveineuse par le pentobarbital sodique (30 mg/kg, i.v.) et la carotide gauche a été isolée. Une seringue hypodermique (27-Gauge) a été insérée en partie proximale de l'artère ainsi qu'en partie distale. Le segment artériel ainsi isolé a été vidé de son sang et lavé par sérum physiologique et la blessure endothéliale a été induite par passage d'un courant d'air sec (240 ml/min pendant 5 minutes). Les aiguilles ont ensuite été retirées, la circulation sanguine a été rétablie et l'incision refermée. Quatorze jours après la lésion, les animaux ont été anesthésiés (pentobarbital sodique, 30 mg/kg, i.v.). Le segment artériel a été isolé, nnçé par le sérum physiologique et incubée pendant 18 heures dans une solution de 10% de formaldéhyde. Les segments artériels ont ensuite été déshydratés dans l'éthanol, inclus dans la parafine, coupés au micotome et colorés à l'hematoxyline-eosine. Les surfaces médiales et intimales ont été quantifiées par analyse d'image (Biocom Imagenia 5000, Lyon, France).

Les résultats montrés au TABLEAU 1 indiquent que le clopidogrel et la simvastatine (5 mg/kg/j) administrés quotidiennement par voie orale chez le lapin inhibent la prolifération des cellules musculaires lisses consécutive à une lésion de l'endothélium par un courant d'air.

Dans tous les cas, l'administration conjointe de clopidogrel et de simvastatine a résulté en un effet synergique significatif *vis-à-vis* de la prolifération des cellules musculaires lisses. C'est-à-dire que lorsque les produits ont été administrés en association, l'effet anttprolifératif obtenu a toujours été supérieur à la simple somme des effets des deux produits testés pns séparément.

**TABLEAU 1**

| Effet des produits seuls ou en association *vis-à-vis* de la prolifération myointimale consécutive à une lésion de l'endothélium vasculaire. | | |
|---|---|---|
| **Produits** | **Doses** | **% d'inhibition de la prolifération myointimale** |
| Clopidogrel | 5 mg/kg/J | 31 ± 4 % |
| Simvastatine | 5 mg/kg/J | 48 ± 7 % |
| Clopidogrel + Simvastatine | 5 + 5 mg/kg/J | 92 ± 9 % |
| Les valeurs du tableau sont des valeurs moyennes ± erreurs standards (n = 10). | | |

Par ailleurs, l'effet antithrombotique de l'association selon l'invention a été mis en évidence dans un test de formation d'un thrombus sur un fil de soie présent dans un shunt artério-veineux implanté entre l'artère carotide et la veine jugulaire du lapin comme décrit par Umetsu *et al*. (Thromb. Haemostas., 1978, *39*, 74-83). Des lapins Néo-Zélandais de 2,5 à 3 kg ont été traités. Les animaux ont été anesthésiés par administration sous-cutanée de pentobarbital sodique (30 mg/kg). Deux tubes en polyéthylène longs de 12 cm (diamètre intérieur : 0,6 mm; diamètre extérieur : 0,9 mm) attachés par une partie centrale de 6 cm de long (diamètre intérieur : 0,9 mm) contenant un fil de soie de 5 cm de long ont été placés entre l'artère carotide droite et la veine jugulaire gauche. La partie centrale du shunt a ensuite été placée puis retirée après 20 minutes de circulation du sang dans le shunt. Le poids du thrombus présent sur le fil de soie a ensuite été déterminé.

De la même manière que dans le cas des effets mesurés *vis-à-vis* de la prolifération des cellules musculaires lisses consécutives à une lésion de l'endothélium par un courant d'air, l'activité antithrombotique du clopidogrel a été potentialisée par une association avec la simvastatine. Dans ces conditions, et comme *vis-à-vis* de la prolifération des cellules musculaires lisses, un effet synergique significatif a été observé. Les résultats obtenus sont repris dans le TABLEAU 2 ci-après.

**TABLEAU 2**

| Effet des produits seuls ou en association *vis-à-vis* de la formation d'un thrombus artériel sur un fil de soie implanté dans un shunt artério-veineux chez le lapin | | |
|---|---|---|
| **Produits** | **Doses** | **% d'inhibition de la thrombose** |
| Simvastatine | 5 mg/kg | 15 ± 4 % |
| Clopidogrel | 5 mg/kg | 34 ± 4 % |
| Clopidogrel + simvastatine | 5 + 5 mg/kg | 72 ± 5 % |
| Les valeurs du tableau sont des valeurs moyennes ± erreurs standards (n = 5). | | |

L'association de la thiénopyridine et de l'inhibiteur de la HMG-CoA réductase est formulée dans les compositions pharmaceutiques utilisables par voie orale ou parentérale, notamment par voie orale, en mélange avec des excipients pharmaceutiques classiques.

Lesdites compositions pharmaceutiques, objet de la présente invention, se présentent de préférence sous forme d'unités de dosage contenant une quantité prédéterminée des principes actifs, telle que précisée ci-dessus. Les formes unitaires d'administration par voie orale comprennent les comprimés, les gélules, les poudres, les granules, les microgranules.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Le principe actif peut être formulé également sous forme de microcapsules, éventuellement avec un ou plusieurs supports ou additifs.

Les principes actifs des associations peuvent être également présenté sous forme de complexe avec une cyclodextrine, par exemple α, β ou γ cyclodextnne, 2-hydroxypropyl-β-cyclodextrine ou méthyl-β-cyclodextrine.

Dans la formulation des associations des principes actifs pour la préparation des composions pharmaceutiques selon la présente invention, on tiendra compte de la nature des Composants (a) et (b) des associations. Le Composant (a), à savoir la thiénopyridine, est de préférence utilisé sous forme de sel d'addition avec des acides pharmaceutiquement acceptables. Par exemple, des Composants (a) préférés sont le chlorhydrate de ticlopidine et l'hydrogénosulfate de clopidogrel qui sont des composés acides.

Normalement, les thiénopyridines, sous forme de leurs sels d'addition avec des acides pharmaceutiquement acceptables, ne sont pas chimiquement incompatibles avec les inhibiteurs de HMG-CoA réductase. Toutefois, certains de ces derniers sont utilisés sous forme de sels avec des métaux alcalins, comme par exemple dans le cas de la pravastatine sodique. Il est donc préférable de maintenir les principes actifs séparés selon des techniques bien connues de la littérature.

Ainsi la forme pharmaceutique contenant l'association de la thiénopyridine et de l'inhibiteur de HMG-CoA réductase peut se présenter, par exemple, comme une gélule. transparente ou opaque, contenant deux comprimés dont l'un renferme la thiénopyridine et l'autre renferme l'inhibiteur de HMG-CoA réductase. Cette présentation a l'avantage d'utiliser les deux principes actifs constituant l'association dans la forme pharmaceutique utilisée couramment, chaque comprimé pouvant être recouvert d'une pellicule qui permette soit la libération immédiate des deux principes actifs, soit une libération programmée en temps différents.

Une autre présentation peut envisager des gélules contenant un mélange de microgranules dont certains contiennent la thiénopyridine et les autres renferment l'inhibiteur de HMG-CoA réductase, lesdits microgranules pouvant être recouverts par une pellicule permettant la libération immédiate ou programmée des principes actifs.

La forme pharmaceutique contenant l'association selon la présente invention peut se présenter comme un comprimé double ou triple couche, plus particulièrement comme comprimés préparés par soumission à plus d'une compression. Le résultat d'une telle forme peut être soit celui d'un comprimé à deux couches, séparées par exemple par une pellicule, soit celui d'un comprimé à l'intérieur d'un autre comprimé, les deux parties étant éventuellement colorées de façon différente.

Une autre forme pharmaceutique de l'association selon la présente invention peut se présenter comme une double gélule constituée par une gélule de gélatine interne contenant un des deux composants et par une gélule de gélatine externe contenant la première gélule et l'autre composant. Dans ce cas, il est préférable que la gélule interne contienne l'inhibiteur de HMG-CoA réductase et l'externe contienne la thiénopyridine. Une forme pharmaceutique de ce type est décnte dans US 5,310,555.

Dans les associations selon la présente invention, les formes pharmaceutiques de la présente invention contienne, de préférence, 250 mg de chlorhydrate de ticlopidine et 20 mg de simvastatine ou de pravastatine sodique 250 mg de chlorhydrate de ticlopidine et 15 mg de simvastatine ou 15 mg de pravastatine sodique; 200 mg de chlorhydrate de ticlopidine et 15 mg de simvastatine ou 15 mg de pravastatine sodique; 175 mg de chlorhydrate de ticlopidine et 20 mg de simvastatine ou de pravastatine sodique; 175 mg de chlorhydrate de ticlopidine et 15 mg de simvastatine ou de pravastatine sodique; 250 mg de chlorydrate de ticlopidine et 10 mg de simvastatine ou de pravastatine sodique; 200 mg de chlorydrate de ticlopidine et 10 mg de simvastatine ou de pravastatine sodique; 175 mg de chlorydrate de ticlopidine et 10 mg de simvastatine ou de pravastatine sodique. Des associations contenant 250 mg de chlorhydrate de tidopidine et 20 mg de simvastatine ou de pravastatine sodique sont également envisageables pour une thérapie d'attaque.

Dans d'autres associations selon la présente invention, les formes pharmaceutiques renferment de préférence 87,5 mg de clopidogrel hydrogénosulfate et 20 mg de simvastatine ou pravastatine sodique; 81,25 mg de clopidogrel hydrogénosulfate et 20 mg de simvastatine ou pravastatine sodique; 87.5 mg de clopidogrel hydrogénosulfate et 15 mg de simvastatine ou pravastatine sodique; 81,25 mg de clopidogrel hydrogénosulfate et 15 mg de simvastatine ou pravastatine sodique; 62,5 mg de clopidogrel hydrogénosulfate et 20 mg de simvastatine ou pravastatine sodique; 62,5 mg de clopidogrel hydrogénosulfate et 15 mg de simvastatine ou pravastatine sodique; 93,75 mg de clopidogrel hydrogénosulfate et 10 mg de simvastatine ou pravastatine sodique; 87,5 mg de clopidogrel hydrogénosulfate et 10 mg de simvastatine ou pravastatine sodique; 81,25 mg de clopidogrel hydrogénosulfate et 10 mg de simvastatine ou pravastatine sodique; 62,5 mg de clopidogrel hydrogénosulfate et 10 mg de simvastatine ou pravastatine sodique. Des associations contenant 87,5 mg de clopidogrel hydrogénosulfate et 20 mg de simvastatine ou pravastatine sodique sont également envisageables pour une thérapie d'attaque.

Les compositions pharmaceutiques de la présente invention sont particulièrement indiquées dans le traitement des états pathologiques tels que les troubles du système cardiovasculaire et cérébrovasculaire comme les troubles tromboemboliques associés à l'athérosclérose ou au diabète tels l'angine instable, l'attaque cérébrale, la resténose après l'angroplastie, l'endartérectomie ou pose de prothèses endovasculaires métalliques, à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires, de pontages aorto-coronariens ou vis à vis de l'angor stable ou instable.

### EXEMPLE 1

### Association ticlopidine/simvastatine (200mg/20mg)

### 1. Formule comprimé double couches

| **Couches n°1** | |
|---|---|
| Ticlopidine (chlorhydrate de) | 200,00 mg |
| Cellulose microcristalline | 69,88 mg |
| Amidon de maïs modifié | 31,20 mg |
| Polyvidone | 6,24 mg |
| Acide citrique | 3,12 mg |
| Acide stéarique | 0,78 mg |
| Stéarate de magnésium | 0,78 mg |

| **Couche n°2** | |
|---|---|
| Simvastatine | 20,00 mg |
| Buthylhydroxyanisole | 0,04 mg |
| Acide ascorbique | 5,00 mg |
| Acide citrique | 2,50 mg |
| Cellulose microcristalline | 10,00 mg |
| Amidon de maïs prégélatinisé | 20,00 mg |
| Lactose | 141, 50 mg |
| Stéarate de magnésium | 1,00 mg |
| Méthylhydroxypropylcellulose | 1,65 mg |
| Hydroxypropylcellulose | 1,65 mg |
| Bioxyde de titanium | 1,50 mg |
| Talc | 0,60 mg |
| Oxyde ferrique jaune | 0,092 mg |
| Oxyde ferrique rouge | 0,023 mg |

### 2. Modes opératoires

◆ Le granulé de ticlopidine est préparé par granulation humide.
◆ Le granulé de simvastatine est préparé par granulation humide.
◆ Les deux granulés sont comprimés sur presse permettant la fabrication des comprimés double couches.

### EXEMPLE 2

### Association clopidogrel/simvastatine (50 mg/10 mg)

### 1. Formule comprimé double couches

| **Couche n°1** | |
|---|---|
| Clopidogrel hydrogénosulfate | 65,00 mg |
| | (soit 50 mg de base) |
| Lactose anahydre | 72,20 mg |
| Amidon de mais modifié | 7,00 mg |
| Macrogel 6000 | 5,00 mg |
| Cellulose microcristalline | 8,60 mg |
| Huile de ricin hydrogénée | 2,20 mg |

| **Couche n°2** | |
|---|---|
| Simvastatine | 10,00 mg |
| Butylhydroxyanisole | 0,02 mg |
| Acide ascorbique | 2,50 mg |
| Acide citrique | 1,75 mg |
| Cellulose microcristalline | 5,00 mg |
| Amidon de maïs pregélatiné | 10,00 mg |
| Lactose | 70,75 mg |
| Stéarate de magnésium | 0,50 mg |
| Méthylhydroxypropylcellulose | 0,825 mg |
| Hydroxypropylcellulose | 0,825 mg |
| Bioxyde de titanium | 0,75 mg |
| Talc | 0,30 mg |
| Oxyde ferrique jaune | 0,046 mg |
| Oxyde ferrique rouge | 0,0115 mg |

### 2. Modes opératoires

◆ Le granulé de Clopidorel est préparé par granulation sèche (compactage).
◆ Le granulé de Simvastatine est préparé par granulation humide.
◆ Les deux granulés sont comprimés sur presse de permettant la fabrication des comprimés double couche.

## Revendications

1. Composition pharmaceutique contenant :
(a) un dérivé de thiénopyridine de formule 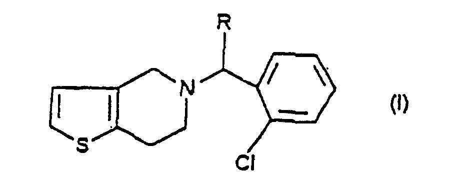 dans laquelle R est l'hydrogène ou un groupe (C₁-C₄)alcoxycarbonyle, ou un de ses sels pharmaceutiquement acceptables; et
(b) un inhibiteur de la HMG-CoA réductase ; en quantité suffisante pour assurer une potentialisation du pouvoir anti-athérogène ou/et de l'activité anti-thrombotique.

2. Composition selon la revendication 1 **caractérisée en ce que** le Composant (a) est présent à une dose entre 10 et 250 mg de principe actif et le Composant (b) est présent à une dose de 2 à 50 mg de principe actif.

3. Composition selon la revendication 1 **caractérisée en ce que** le dérivé de thiénopyridine est le chlorhydrate de ticlopidine.

4. Composition selon la revendication 3 **caractérisée en ce que** la quantité de chlorhydrate de ticlopidine dans une unité de dosage est de 100 à 250 mg.

5. Composition selon la revendication 1 **caractérisée en ce que** le dérivé de thiénopyridine est l'hydrogénosulfate de clopidogrel.

6. Composition selon la revendication 5 **caractérisée en ce que** la quantité d'hydrogénosulfate dans l'unité de dosage est de 10 à 75 mg (calculés en base libre).

7. Composition selon la revendication 1 **caractérisée en ce que** l'inhibiteur de HMG-CoA réductase est un composé choisi parmi :
(i) les dérivés de naphtalène de formule (II) 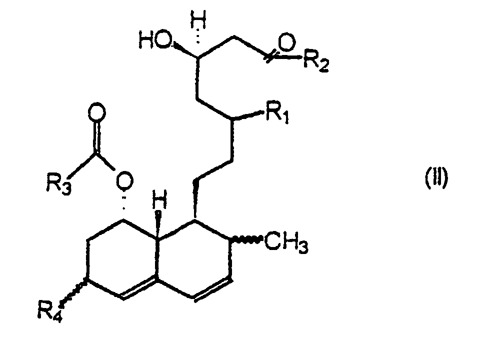 dans laquelle R₁ et R₂ sont un groupe hydroxy ou bien, ensemble forment un atome d'oxygène, R₃ est un groupe (C₁-C₁₀)alkyle, (C₃-C₁₀)cycloalkyle, (C₂-C₁₀)alkényle, phényle ou phényl(C₁-C₃)alkyle et R₄ est l'hydrogène ou un groupe méthyle ou hydroxyle;
(ii) les sels pharmaceutiquement acceptables des composés de formule (II) dans laquelle R ₁ et R₂ sont hydroxy;
(iii) les dérivés de l'indole de formule (III) 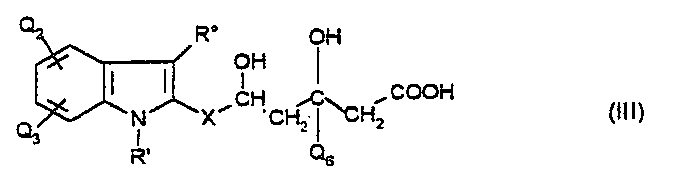 dans laquelle
- un des R° et R' est un groupe de structure 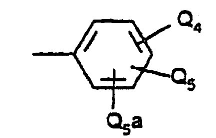
dans laquelle Q₄ est un atome d'hydrogène, de chlore ou de fluor ou un groupe (C₁-C₄)alkyle, (C₁-C₄)alcoxy, (mais autre que t-butoxy), trifluorométhyle, phénoxy ou benzyloxy, Q₅ est un atome d'hydrogène, de chlore, de fluor ou un groupe phénoxy ou benzyloxy, Q₅ₐ est un atome d'hydrogène, de chlore, de fluor ou un groupe méthyle, éthyle, méthoxy ou éthoxy; et l'autre des R° et R' est un un groupe (C₁-C₆)alkyle primaire ou secondaire, (C₃-C₆)cycloalkyle, benzyle, 2-phényléthyle ou 3-phénylpropyle;
- Q₂ est un atome d'hydrogène, de fluor, de chlore ou un groupe (C₁-C₄)alkyle, (C₃-C₆)cycloalkyle, (C₁-C₄)alcoxy (mais, autre que t-butoxy), trifluorométhyle, phénoxy ou benzyloxy;
- Q₃ est un atome d'hydrogène, de chlore, de fluor ou un groupe (C₁-C₃)alkyle, (C₁-C₃)alcoxy, phénoxy ou benzyloxy;
- X est un groupe méthylène, éthylène ou 1,3-propylène;
- Q₆ est un atome d'hydrogène ou un groupe (C₁-C₃)alkyle;
avec la limitation que
(1) Q₅ et Q₅ₐ sont l'hydrogène lorsque R₄ est l'hydrogène,
(2) Q₅ₐ est l'hydrogène lorsque Q₅ est l'hydrogène,
(3) Q₄ et Q₃ ne sont pas en même temps un groupe trifluorométhyle, phénoxy ou benzyloxy,
(4) Q₃ est l'hydrogène lorsque Q₂ est l'hydrogène,
(5) Q₂ et Q₃ ne sont pas en même temps un groupe trifluorométhyle, phénoxy ou benzyloxy;
(iv) les esters pharmaceutiquement acceptables des composés de formule (III),
(v) les sels pharmaceutiquement acceptables des composés de formule (III),
(vi) les δ-lactones des composés de formule (III),
(vii) les dérivés du tétrazole de formule (IV) 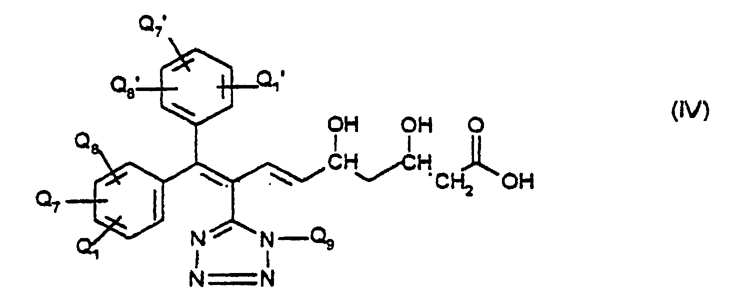 dans laquelle
- Q₁ et Q₁' sont l'hydrogène, un halogène ou un groupe (C₁-C₄)alkyle, (C₁-C₄)-alcoxy ou trifluorométhyle;
- Q₇, Q₇', Q₈ et Q₈' sont l'hydrogène, un halogène ou un groupe (C₁-C₄)alkyle, (C₁-C₄)alcoxy;
- Q₉ est l'hydrogène ou un groupe (C₁-C₄)alkyle, (C₁-C₄)alcoxyalkyle ou (2-méthoxyéthoxy)méthyle;
(viii) les sels pharmaceutiquement acceptables des composés de formule (IV),
(ix) les δ-lactones des composés de formule (IV),
(x) les dérivés de pyridine de formule (V) 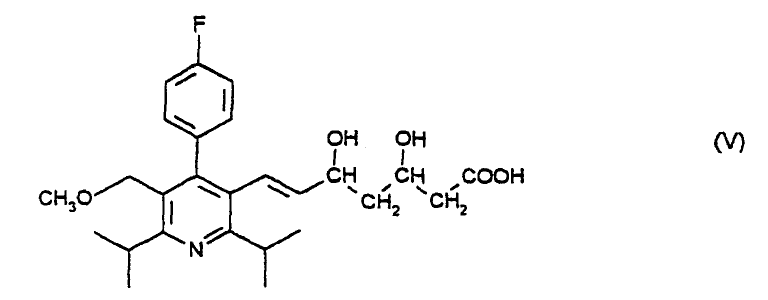
(xi) les sels pharmaceutiquement acceptables des composés de formule (V)
(xii) les δ-lactones des composés de formule (V),
(xiii) les dérivés du pyrrole de formule (VI) 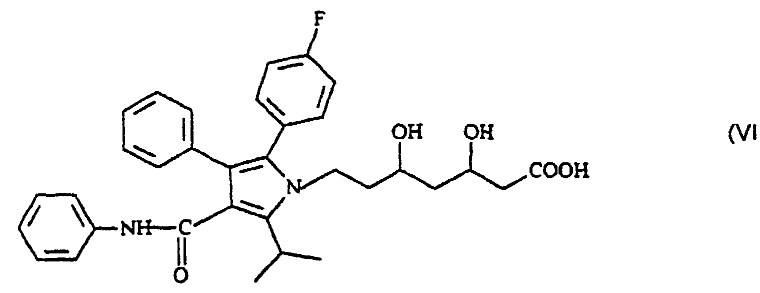
(xiv) les sels pharmaceutiquement acceptables des composés de formule (VI),
(xv) les δ-lactones des composés de formule (VI).

8. Composition selon la revendication 7 **caractérisée en ce que** l'inhibiteur de HMG-CoA réductase est choisi parmi la simvastatine, la pravastatine sodique, la mévastatine, la lovastatine, la cérivastatine, l'atorvastatine, un dérivé de l'indole de formule (III) dans laquelle R° est 4-fluorophényle, R' est isopropyle, X est éthylène et Q₂, Q₃ et Q₆ sont l'hydrogène dans sa forme (E) racémique ou optiquement active, les sels pharmaceutiquement acceptables dudit dérivé de l'indole, un dérivé du tétrazole de formule (IV) dans laquelle Q₁ et Q₁' sont chacun un atome de fluor, Q₇, Q₇', Q₈ et Q₈' sont l'hydrogène, dans sa forme (E) de configuration (βR), (δS) et les sels pharmaceutiquement acceptables dudit dérivé de tétrazole.

9. Composition selon la revendication 2 **caractérisée en ce que** le Composant (a) est le chlorhydrate de ticlopidine et le Composant (b) est choisi parmi la simvastatine et la pravastatine sodique.

10. Composition selon la revendication 9 **caractérisée en ce qu'**elle renferme de 1.00 à 250 mg de chlorhydrate de ticlopidine et de 10 à 40 mg de simvastatine ou de pravastatine sodique.

11. Composition selon la revendication 2 **caractérisée en ce que** le Composant (a) est l'hydrogénosulfate de clopidogrel et le Composant (b) est choisi parmi la simvastatine ou la pravastatine sodique.

12. Composition selon la revendication 11 **caractérisée en ce qu'**elle renferme de 10 à 75 mg (calculé en base libre) de clopidogrel hydrogénosulfate et de 10 à 40 mg de simvastatine ou de pravastatine sodique.

13. Utilisation pour la fabrication d'un médicament antithrombotique d'une association :
(a) d'un dérivé de thiénopyridine de formule 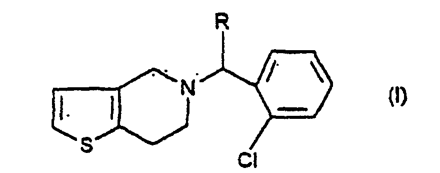 dans laquelle R est l'hydrogène ou un groupe (C₁-C₄)alcoxycarbonyle, ou un de ses sels pharmaceutiquement acceptables; et
(b) d'un inhibiteur de la HMG-CoA-réductase.

14. Utilisation pour la fabrication d'un médicament antiathérogène d'une association :
(a) d'un dérivé de thiénopyridine de formule 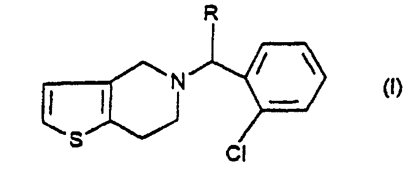 dans laquelle R est l'hydrogène ou un groupe (C₁-C₄)alcoxycarbonyle, ou un de ses sels pharmaceutiquement acceptables; et
(b) d'un inhibiteur de la HMG-CoA-réductase.

## Claims

1. Pharmaceutical composition containing:
(a) a thienopyridine derivative of formula 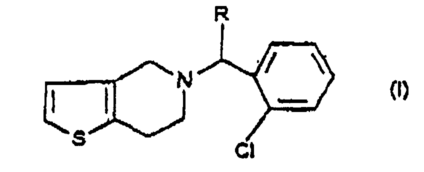 wherein R is hydrogen or a (C₁-C₄) alkoxy carbonyl group, or one of the pharmaceutically acceptable salts thereof; and
(b) an inhibitor of HMG-CoA reductase;
in a sufficient amount to ensure potentiation of the anti-atherogenic and/or anti-thrombotic activity.

2. Composition according to claim 1, **characterised in that** component (a) is present in an amount of between 10 and 250 mg of active ingredient and component (b) is present in an amount of 2 to 50 mg of active ingredient.

3. Composition according to claim 1, **characterised in that** the thienopyridine derivative is ticlopidine hydrochloride.

4. Composition according to claim 3, **characterised in that** the amount of ticlopidine hydrochloride in a dosage unit is from 100 to 250 mg.

5. Composition according to claim 1, **characterised in that** the thienopyridine derivative is clopidogrel hydrogen sulphate.

6. Composition according to claim 5, **characterised in that** the quantity of hydrogen sulphate in the dosage unit is from 10 to 75 mg (calculated as free base).

7. Composition according to claim 1, **characterised in that** the HMG-CoA reductase inhibitor is a compound selected from among:
(i) the naphthalene derivatives of formula (II) 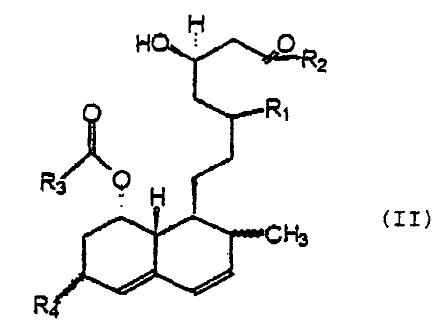 wherein R₁ and R₂ are a hydroxy group or together form an oxygen atom, R₃ is a C₁-C₁₀ alkyl, C₃-C₁₀ cycloalkyl, C₂-C₁₀ alkenyl, phenyl or phenyl C₁-C₃ alkyl group and R₄ is hydrogen or a methyl dr hydroxyl group;
(ii) the pharmaceutically acceptable salts of he compounds of formula II wherein R₁ and R₂ are hydroxy;
(iii) the indole derivatives of formula III 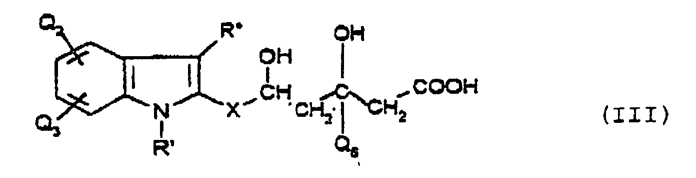 wherein
one of R° and R' is a group of the structure 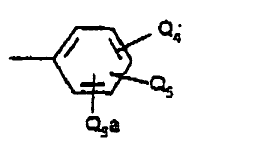
wherein Q₄ is a hydrogen, chlorine or fluorine atom or a C₁-C₄ alkyl, C₁-C₄ alkoxy (other than t-butoxy) , trifluoromethyl, phenoxy or benzyloxy group, Q₅ is a hydrogen, chlorine, fluorine atom or.a phenoxy or benzyloxy group, Q₅ₐ is a hydrogen, chlorine or fluorine atom or a methyl, ethyl, methoxy or ethoxy group: and the other one of R° and R' is a primary or secondary C₁-C₆ alkyl, C₃-C₆ cycloalkyl, benzyl, 2-phenylethyl or 3-phenylpropyl group;
Q₂ is a hydrogen fluorine or chlorine atom of a C₁-C₄ alkyl, C₃-C₆ cycloalkyl, C₁-C₄ alkoxy (other than t-butoxy), trifluoromethyl, phenoxy or benzyloxy group;
Q₃ is a hydrogen, chlorine or fluorine atom or a C₁-C₃ alkyl, C₁-C₃ alkoxy, phenoxy or benxyloxy group;
X is a methylene, ethylene or 1,3-propylene group;
Q₆ is a hydrogen atom or a C₁-C₃ alkyl group;
with the proviso that
(1) Q₅ and Q₅ₐ denote hydrogen when R₄ is hydrogen,
(2) Q₅ₐ is hydrogen when Q₅ is hydrogen,
(3) Q₄ and Q₃ are not simultaneously a trifluoromethyl, phenoxy or benzyloxy group,
(4) Q₃ is hydrogen when Q₂ is hydrogen,
(5) Q₂ and Q₃ are not simultaneously a trifluoromethyl, phenoxy or benzyloxy group;
(iv) the pharmaceutically acceptable eaters of the compounds of formula III,
(v) the pharmaceutically acceptable salts of the compounds of formula III,
(vi) the δ-lactones of the compounds of formula III,
(vii) the tetrazole derivatives of formula IV 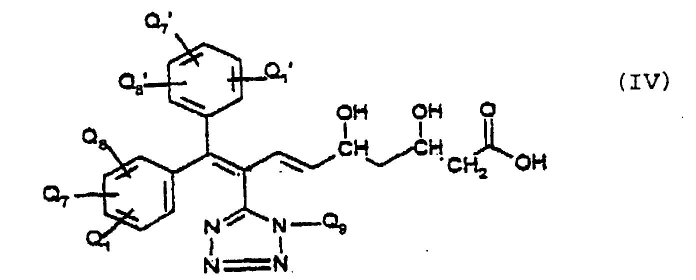 wherein
Q₁ and Q₁' are hydrogen a halogen or a C₁-C₄ alkyl, C₁-C₄ alkoxy or trifluoromethyl group;
Q₇, Q₇', Q₈ and Q₈' are hydrogen, a halogen or a C₁-C₄ alkyl or C₁-C₄ alkoxy group;
Q₉ is hydrogen or a C₁-C₄ alkyl, C₁-C₄ alkoxy alkyl or 2-methoxyethoxy methyl group;
(viii) the pharmaceutically acceptable salts of the compounds of formula IV,
(ix) the δ-lactones of the compounds of formula IV,
(x) the pyridine derivatives of formula V 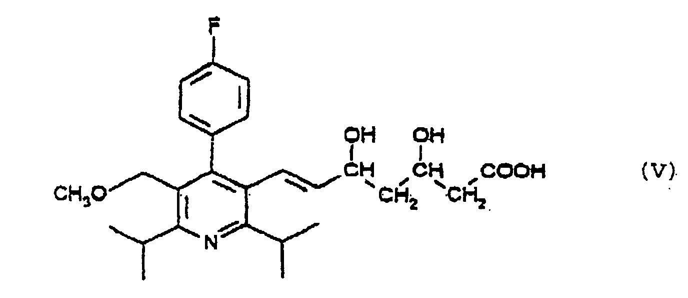
(xi) the pharmaceutically acceptable salts of the compounds of formula V,
(xi) the δ-lactones of the compounds of formula V,
(xiii)the pyrrole derivatives of formula VI 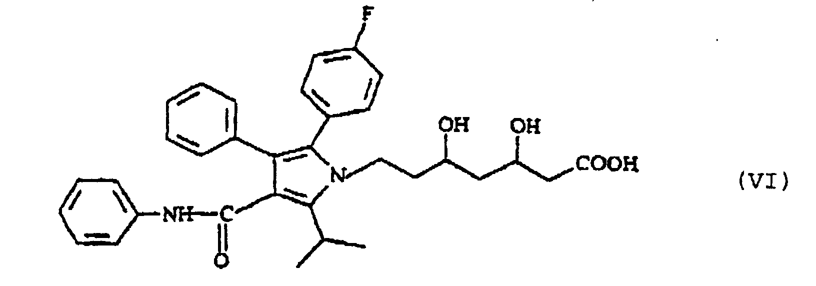
(xiv)the pharmaceutically acceptable salts of the compounds of formula VI,
(xv) the δ-lactones of the compounds of formula VI.

8. Composition according to claim 7, **characterised in that** the HMG-CoA reductase inhibitor is selected from among simvastatin, sodium pravastatin, mevastatin, lovastatin, cerivastatin, atorvastatin, an indole derivative of formula III wherein R° is 4-fluorophenyl, R' is isopropyl, X is ethylene and Q₂, Q₃ and Q₆ are hydrogen in its racemic (E) or optically active form, the pharmaceutically acceptable salts of said indole derivative, a tetrazole derivative or formula IV wherein Q₁ and Q₁' each denote a fluorine atom, Q₇, Q₇', Q₈ and Q₈' denote hydrogen, in its (E) form of (βR), (δS) configuration and the pharmaceutically acceptable salts of said tetrazole derivative.

9. Composition according to claim 2, **characterised in that** component (a) is ticlopidine hydrochloride and component (b) is selected from among simvastatin and sodium pravastatin.

10. Composition according to claim 9, **characterised in that** it contains from 100 to 250 mg of ticlopidine hydrochloride and from 10 to 40 mg of simvastatin or sodium pravastatin.

11. Composition according to claim 2, **characterised in that** component (a) is clopidogrel hydrogen sulphate and component (b) is selected from among simvastatin and sodium pravastatin.

12. Composition according to claim 11, **characterised in that** it contains from 10 to 75 mg (calculated as free base) of clopidogrel hydrogen sulphate and from 10 to 40 mg of simvastatin or sodium pravastatin.

13. Use of a combination of:
(a) a thienopyridine derivative of formula 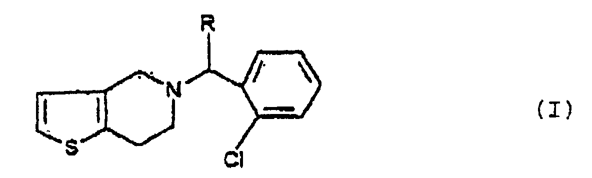 wherein R is hydrogen or a C₁-C₄ alkoxycarbonyl group, or one of the pharmaceutically acceptable salts thereof;
and
(b) an HMG-CoA reductase inhibitor;
for the production of an antithrombotic drug.

14. Use of a combination of:
(a) a thienopyridine derivative of formula 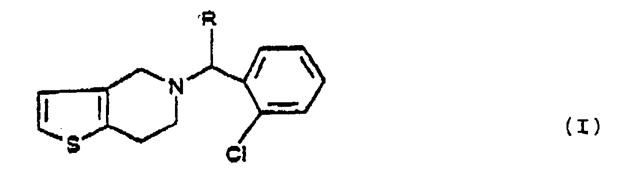 wherein R is hydrogen or a C₁-C₄ alkoxycarbonyl group, or one of the pharmaceutically acceptable salts thereof;
and
(b) an HMG-CoA reductase inhibitor;
for the manufacture of an anti-atherogenic drug.

## Patentansprüche

1. Pharmazeutische Zubereitung enthaltend;
(a) ein Thienopyridin-Derivat der Formel 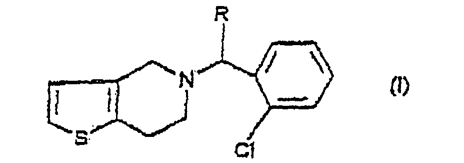 in der R Wasserstoff oder eine (C₁-C₄)-Alkoxycarbonylgruppe bedeutet, oder eines seiner pharmazeutisch annehmbaren Salze; und
(b) einen Inhibitor der HMG-CoA-Reduktase; in einer Menge, die dazu ausreicht, eine Potenzierung der anti-atherogenen Wirkung und/oder der anti-thrombotischen Wirkung sicherzustellen.

2. Zubereitung nach Anspruch 1, **dadurch gekennzeichnet, daß** der Bestandteil (a) in einer Dosis zwischen 10 und 250 mg des Wirkstoffs und der Bestandteil (b) in einer Dosis von 2 bis 50 mg des Wirkstoffs vorhanden sind.

3. Zubereitung nach Anspruch 1, **dadurch gekennzeichnet, daß** das Thienopyridin-Derivat Ticlopidin-Hydrochlorid ist.

4. Zubereitung nach Anspruch 3, **dadurch gekennzeichnet, daß** die Menge des Ticlopidin-Hydrochlorids in einer Dosiseinheit 100 bis 250 mg beträgt.

5. Zubereitung nach Anspruch 1, **dadurch gekennzeichnet, daß** das Thienopyridin-Derivat Clopidogrel-Hydrogensulfat ist.

6. Zubereitung nach Anspruch 5, **dadurch gekennzeichnet. daß** die Menge des Hydrogensulfats in der Dosiseinheit 10 bis 75 mg (berechnet als freie Base) beträgt.

7. Zubereitung nach Anspruch 1, **dadurch gekennzeichnet, daß** der Inhibitor der HMG-CoA-Reduktase eine Verbindung ist, ausgewählt aus:
(i) Naphthalin-Derivaten der Formel (II) 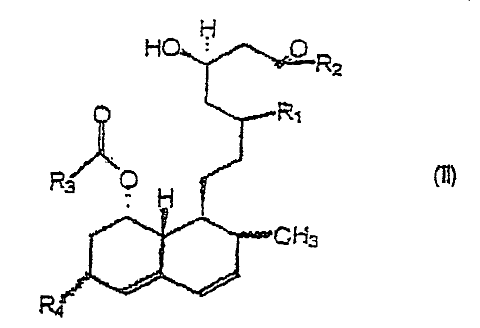 in der R₁ und R₂ Hydroxygruppen bedeuten oder gemeinsam ein Sauerstoffatom bilden, R₃ eine (C₁-C₁₀)-Alkylgruppe, (C₃-C₁₀)-Cycloalkylgruppe, (C₂-C₁₀)-Alkenylgruppe, Phenylgruppe oder Phenyl-(C₁-C₃)-alkylgruppe darstellt und R₄ Wasserstoff oder eine Methyl- oder Hydroxygruppe bedeutet;
(ii) den pharmazeutisch annehmbaren Salzen der Verbindungen der Formel (II), in der R₁ und R₂ Hydroxygruppen bedeuten;
(iii) Indol-Derivaten der Formel (III) 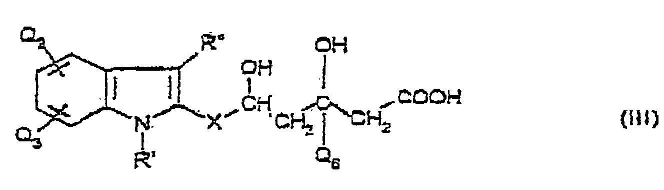 in der
- eine der Gruppen R° und R' eine Gruppe der Formel 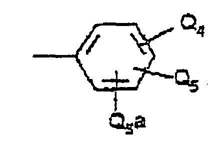 darstellt, in der Q₄ ein Wasserstoffatom, ein Chloratom oder ein Fluoratom oder eine (C₁-C₄)-Alkylgruppe, (C₁-C₄)-Alkoxygruppe (die Jedoch von der tert.-Butoxygruppe verschieden ist), eine Trifluormethylgruppe, Phenoxygruppe oder Benzyloxygruppe darstellt, Q₅ ein Wasserstoffatom, ein Chloratom, ein Fluoratom oder eine Phenoxy- oder Benzyloxygruppe bedeutet, Q₅ₐ ein Wasserstoffatom, ein Chloratom, ein Fluoratom oder eine Methyl-, Ethyl-, Methoxy- oder Ethoxygruppe darstellt;
und die andere der Gruppen R° und R' eine primäre oder sekundäre (C₁-C₆)-Alkylgruppe, (C₃-C₆)-Cycloalkylgruppe, Benzylgruppe, 2-Phenylethylgruppe oder 3-Phenylpropylgruppe bedeutet;
- Q₂ ein Wasserstoffatom, Fluoratom, Chloratom oder eine (C₁-C₄)-Alkylgruppe, (C₃-C₆)-Cycloalkylgruppe, (C₁-C₄)-Alkoxygruppe (die jedoch von der tert.-Butoxygruppe verschieden ist), Trifluormethylgruppe, Phenoxygruppe oder Benzyloxygruppe darstellt;
- Q₃ ein Wasserstoffatom, Chloratom, Fluoratom oder eine (C₁-C₃)-Alkylgruppe, (C₁-C₃)-Alkoxygruppe, Phenoxygruppe oder Benzyloxygruppe darstellt;
- X eine Methylen-, Ethylen- oder 1,3-Propylengruppe bedeutet;
- Q₆ ein Wasserstoffatom oder eine (C₁-C₃)-Alkylgruppe darstellt;
mit der Maßgabe, daß
(1) Q₅ und Q₅ₐ Wasserstoff bedeuten, wenn R₄ Wasserstoff bedeutet,
(2) Q₅ₐ Wasserstoff bedeutet, wenn Q₅ Wasserstoff bedeutet,
(3) Q₄ und Q₃ nicht gleichzeitig eine Trifluormethyl-, Phenoxy- oder Benzyloxygruppe bedeuten,
(4) Q₃ Wasserstoff bedeutet, wenn Q₂ Wasserstoff bedeutet,
(5) Q₂ und Q₃ nicht gleichzeitig eine Trifluormethyl-, Phenoxy- oder Benzyloxygruppe bedeuten;
(iv) den pharmazeutisch annehmbaren Estern der Verbindungen der Formel (III),
(v) den pharmazeutisch annehmbaren Salzen der Verbindungen der Formel (III),
(vi) den δ-Lactonen der Verbindungen der Formel (III),
(vii) Tetrazol-Derivaten der Formel (IV) 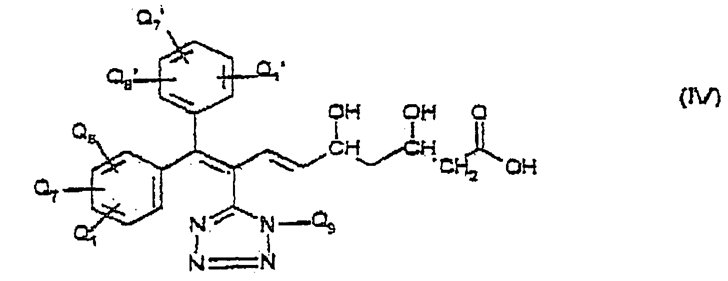 in der
- Q₁ und Q₁' Wasserstoff, Halogen oder (C₁-C₄)-Alkyl-, (C₁-C₄)-Alkoxy- oder Trifluormethylgruppen bedeuten:
- Q₇, Q₇', Q₈ und Q₈' Wasserstoff, Halogen oder (C₁-C₄)-Alkylgruppen oder (C₁-C₄)-Alkoxygruppen darstellen;
- Q₉ Wasserstoff oder eine (C₁-C₄)-Alkylgruppe, (C₁-C₄)-Alkoxyalkylgruppe oder (2-Methoxyethoxy)-methylgruppe bedeutet;
(viii) den pharmazeutisch annehmbaren Salzen der Verbindungen der Formel (IV),
(ix) den δ-Lactonen der Verbindungen der Formel (IV),
(x) Pyridin-Derivaten der Formel (V) 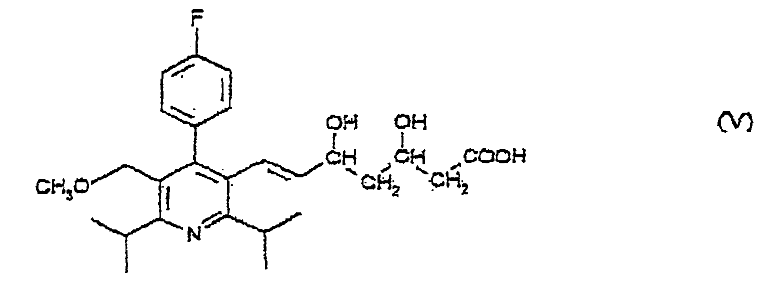
(xi) den pharmazeutisch annehmbaren Salzen der Verbindungen der Formel (V),
(xii) den δ-Lactonen der Verbindungen der Formel (V),
(xiii) Pyrrol-Derivaten der Formel (VI) 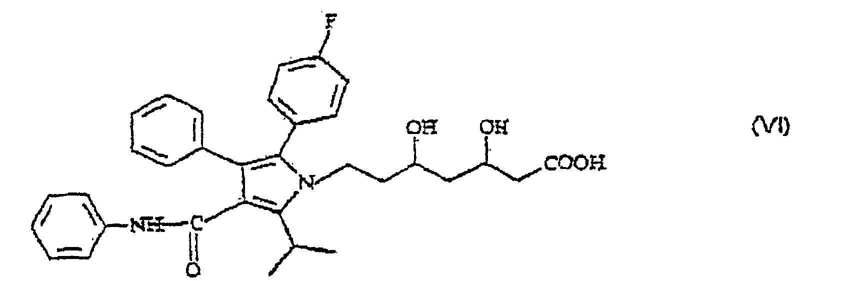
(xiv) den pharmazeutisch annehmbaren Salzen der Verbindungen der Formel (VI), und
(xv) den δ-Lactonen der Verbindungen der Formel (VI).

8. Zubereitung nach Anspruch 7, **dadurch gekennzeichnet, daß** der Inhibitor der HMG-CoA-Reduktase ausgewählt ist aus Simvastatin, Natrium-Pravastatin, Mevastatin, Lovastatin, Cerivastatin, Atorvastatin, einem Indol-Derivat der Formel (III), in der R° 4-Fluorphenyl, R' Isopropyl, X Ethylen und Q₂, Q₃ und Q₆ Wasserstoff bedeuten, in der racemischen oder optisch aktiven Form (E), den pharmazeutisch annehmbaren Salzen dieses Indol-Derivats, einem Tetrazol-Derivat der Formel (IV), in der Q₁ und Q₁' jeweils Fluoratome, Q₇, Q₇', Q₈ und Q₈' Wasserstoff bedeuten, in der Form (E) der Konfiguration (βR), (δS) und den pharmazeutisch annehmbaren Salzen dieses Tetrazol-Derivats.

9. Zubereitung nach Anspruch 2, **dadurch gekennzeichnet, daß** der Bestandteil (a) Ticlopidin-Hydrochlorid ist und der Bestandteil (b) aus Simvastatin und Natrium-Pravastatin ausgewählt ist.

10. Zubereitung nach Anspruch 9, **dadurch gekennzeichnet, daß** sie 100 bis 250 mg Ticlopidin-Hydrochlorid und 10 bis 40 mg Simvastatin oder Natrium-Pravastatin enthält.

11. Zubereitung nach Anspruch 2, **dadurch gekennzeichnet, daß** der Bestandteil (a) Clopidogrel-Hydrogensulfat ist und der Bestandteil (b) aus Simvastatin und Natrium-Pravastatin ausgewählt ist.

12. Zubereitung nach Anspruch 11, **dadurch gekennzeichnet, daß** sie 10 bis 75 mg (als freie Base gerechnet) Clopidogrel-Hydrogensulfat und 10 bis 40 mg Simvastatin oder Natrium-Pravastatin enthält.

13. Verwendung einer Kombination aus:
(a) einem Thienopyridin-Derivat der Formel 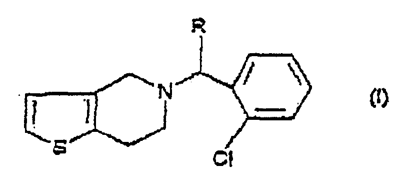 in der R Wasserstoff oder eine (C₁-C₄)-Alkoxycarbonylgruppe bedeutet, oder einem seiner pharmazeutisch annehmbaren Salze; und
(b) einem Inhibitor der HMG-CoA-Reduktase
für die Herstellung eines anti-thrombotischen Arzneimittels.

14. Verwendung einer Kombination aus:
(a) einem Thienopyridin-Derivat der Formel 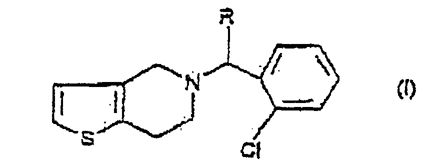 in der R Wasserstoff oder eine (C₁-C₄)-Alkoxycarbonylgruppe bedeutet, oder eines seiner pharmazeutisch annehmbaren Salze; und
(b) einem Inhibitor der HMG-CoA-Reduktase
für die Herstellung eines anti-atherogenen Arzneimittels.
